# EUROPÄISCHE PATENTSCHRIFT

(11) **EP 1 355 599 B1**
(45) Veröffentlichungstag und Bekanntmachung des Hinweises auf die Patenterteilung: **06.04.2005**
(21) Anmeldenummer: 02715958.1
(22) Anmeldetag: 16.01.2002
(51) Int. Cl.: A61F 7/08

(54) **GESCHICHTETE EINRICHTUNG ZUR ABGABE CHEMISCH-/PHYSIKALISCHER PARAMETER**
LAMINATE DEVICE FOR THE OUTPUT OF CHEMICAL/PHYSICAL PARAMETERS
DISPOSITIF EN COUCHES DE SORTIE DE PARAMETRES PHYSICO-CHIMIQUES

(30) Priorität: 30.01.2001 AT 1492001
(43) Veröffentlichungstag der Anmeldung: 29.10.2003
(73) Patentinhaber: Gluderer, Lothar, Erich, A-6130 Schwaz (AT)
(72) Erfinder: Gluderer, Lothar, Erich, A-6130 Schwaz (AT)
(74) Vertreter: Sonn & Partner Patentanwälte
(86) Internationale Anmeldenummer: PCT/AT2002/000013
(87) Internationale Veröffentlichungsnummer: WO 2002/060363

(56) Entgegenhaltungen:
- WO-A-00/67685
- WO-A-99/44552
- US-A- 5 411 494
- US-A- 5 941 907

## Beschreibung

Die Erfindung betrifft eine Einrichtung zur Abgabe chemisch-/physikalischer Parameter und zum Anlegen an Körper oder Teilen davon, wobei die Einrichtung eine Auflage mit wenigstens zwei Schichten aufweist und der zwischen den Schichten bestehende Raum in mindestens zwei Kammern und/oder Kanälen unterteilt ist, welche mit gasförmigen und/oder flüssigen Medien zur Abgabe der chemisch-/physikalischen Parameter befüllbar sind, und die Einrichtung weiters zumindest eine Steuer- bzw. Regeleinrichtung zur Steuerung bzw. Regelung von Parametern, wie beispielsweise Durchflussmenge, Temperatur, Druck oder dergleichen, der in dem Raum befindlichen Medien aufweist, die mit der Auflage verbunden ist, wobei Biosensoren zur Erfassung von Körperparametern, wie beispielsweise Temperatur, EKG oder dergleichen, mit der Steuer- und Regeleinrichtung verbunden sind.

Äußere Heilanwendungen, wie Wärme-, Kälte-, Moor-, Schlammpackungen oder etwa Heu-, Thalassobäder, zählen heutzutage zu beliebten Behandlungsformen und finden in zahlreichen Zentren, wie Krankenhäuser, Rekovaleszenzzentren, Vitalbäder etc., häufige Verwendung. Daher besteht ein Bestreben derartiger Therapieformen zu verbessern. Insbesondere im Bereich der Lokalanwendungen, wie dies Packungen darstellen, gilt es, kostengünstigere und einfachere Vorrichtungen zur Verfügung zu stellen, welche effektiver und leichter applizierbar sind und gleichzeitig eine erhöhte heilende bzw. wohltuende Wirkung erzielen.

Eine dieser Verbesserungen ist aus der EP 144 571 bekannt, welche eine Vorrichtung zum Anlegen von Wärmepackungen offenbart. Diese können mit einem anliegenden Wärmespeicher in Kontakt gebracht werden, wobei Wärmepackungen an den zu behandelnden Körperstellen angelegt und mit einer die Wärmeübertragung dämmenden Isolierschicht versehen werden. Eine Befestigung wird dann mittels unter Druck stehender Andrückfolie erzielt, welche mit einem beheizbaren bzw. aufgeheizten und als Wärmespeicher dienenden flüssigen, breiigen oder gasförmigen Medium druckbeaufschlagbar sind. Die Verwendung einer derartigen Andrückfolie dient nicht nur dem festen Anlegen der Wärmepackungen mitsamt der Isolierschicht, sondern auch durch das sowohl aufheizbare als auch umwälzbare Medium in der Andrückfolie als Temperaturabgabe.

Die Andrückfolie kann an der Oberseite einer Wanne gespannt und heb- bzw. senkbar angeordnet sein, wobei in die Wanne selbst (oder in einen Formballon in der Wanne) aufheizbares Medium eingegeben werden kann und direkt oder indirekt ergänzend als Wärmeabgabe dient. Dabei können die Formballons auch zusätzlich als Andrückfolie auf den in die Wanne herabgesenkten Patienten wirken. Der Formballon ist unterteilbar, so dass u.a. auch Seitenballons entstehen, die weiters durch zusätzliche interne Trennwände in entsprechend separat mit Wärmemedium durchströmte Sektoren unterteilt sein können. Eine Umwälzung und Beheizung des Formballons und/oder Seitenballons wird durch Zu- und Ablauf über einen Wärmetauscher bzw. über eine Heizung und eine Pumpe erreicht. Gegebenenfalls ist der Formballon durch eine Rücklaufleitung mit einem Rücklaufdrosselventil mit den Seitenballons verbunden.

Allerdings müssen für eine ordnungsgemäße und zielführende Wärmebehandlung mittels dieser Vorrichtung mehrere Folien bzw. Schichten nacheinander einzeln auf den zu behandelnden Körper bzw. Körperteilen angelegt oder aufgebracht werden. Ein einfaches und rasches Anbringen der Packungen ist demnach nur schwer möglich, was oft von Nachteil für den zu behandelnden Patienten ist, da er für eine längere Zeit in einer für ihn unbequemen Position oder Stellung verweilen muss. Weiters ist eine direkte Applikation der Behandlungsmittel, wie Schlamm, Moor oder dergl. durch die Wärmepackungen, welche auf die zu behandelnden Körperstellen aufgelegt werden, zwar möglich, diese Wärmepackungen sind jedoch in keiner Weise dosierbar oder steuerbar. Außerdem können nur Wärmepackungen im Sinne von festen bzw. viskosen Stoffen, wie Moor oder Schlamm, aufgetragen werden. Eine direkte Abgabe von flüssigen oder gasförmigen Medien ist hier nicht ermöglicht.

Ein weiterer Nachteil der genannten Vorrichtung ergibt sich durch das Anbringen von Druck und/oder Temperatur, welches zwar an sich problemlos durchgeführt werden kann, allerdings eine Steuerung desselben nur in einem begrenzten Rahmen ermöglicht. Das bedeutet, dass ein Helfer oder Arzt diese Steuerung durchführen muss, was allerdings dazu führt, dass das Druck- bzw. Temperaturverhalten nicht optimal an die Bedürfnisse des Patienten angepasst werden kann, da der Patient selbst weder aktiv noch passiv Einfluss auf den Anpressdruck bzw. Temperatur hat.

Die WO 99/44552 Al beschreibt eine Vorrichtung und ein Verfahren zur Kontrolle der Körpertemperatur eines lebenden Körpers mit einem Wärmeaustauscher zur Entfernung von Wärme oder Übertragung von Wärme auf den Körper. Eine Bedeckung zum Bedecken des Körpers ist durch zwei flexible Schichten gebildet, die mittels einzelnen, unterbrochenen Trennwänden in miteinander kommunizierende Bereiche unterteilt ist, durch welche ein Fluid, wie Wasser, Alkohol, Öl oder dergl., geleitet wird. In einer Ausführungsform zeigt die Bedeckung Öffnungen für einen Zugang des Fluids zur Haut des Körpers. Sensoren bzw. Messfühler oder andere Messeinrichtungen sind zur Erfassung der Körpertemperatur, des Blutdrucks oder Hautwiderstandes verfügbar. Eine Steuer- und Regeleinrichtung zum Einstellen der Eigenschaften des Fluids ist ebenso vorgesehen wie Einrichtungen zum Empfangen und Senden von Daten für die Kontrolle der Körpertemperatur des lebenden Körpers. Mittels dieser Vorrichtung kann der Patient zwar indirekt über die Messfühler bzw. der Steuer- und Regeleinrichtung auf die Temperatur Einfluss nehmen, allerdings ist diese Vorrichtung relativ unflexibel. Die Vorrichtung ist lediglich für den Temperaturaustausch zwischen Körper und Bedeckung geeignet, wobei die Temperaturkontrolle selbst nur für die gesamte Bedeckung bzw. des bedeckten Körpers möglich ist, d.h. auf lokale, ausgewählte Körperbereiche kann kein Einfluß genommen werden.

Es ist nun Ziel der Erfindung, hier Abhilfe zu schaffen und eine Einrichtung der eingangs angeführten Art vorzusehen, welche einfach handhabbar ist, und mittels welcher mehrere chemisch/physikalische Parameter, wie Dampf, Feuchtigkeit, aber auch Druck und dergl., abgegeben werden können. Des weiteren soll die Einrichtung hinsichtlich der Abgabe flexibel sein, sodass größere Bereiche des Körpers, aber auch lokal begrenzte Körperstellen beeinflußbar sind.

Die erfindungsgemäße Einrichtung der eingangs angeführten Art ist dadurch gekennzeichnet, dass die Kammern und/oder Kanäle unabhängig voneinander mit den Medien befüllbar sind, und dass die Medien in den jeweiligen Kammern und/oder Kanälen über die Steuer- bzw. Regeleinrichtung in Abhängigkeit der mittels der Biosensoren erfassten Körperparameter steuer- bzw. regelbar sind.

Gemäß der vorliegenden Erfindung wird nicht nur eine unmittelbare Abgabe von chemisch-/physikalischen Parametern, sondern auch gleichzeitig eine Druckbeaufschlagung, welche die Auflage in engem, direkten Kontakt zum Körper hält, realisiert. Die chemisch-/physikalischen Parameter umfassen die Abgabe gasförmiger und/oder flüssiger Medien, wie Dampf, Flüssigkeit, beispielsweise mit Essenzen oder Duftstoffen (Aromatherapie) versetztes Wasser oder viskose Stoffe, aber auch die Abgabe von Druck, Temperatur und/oder dergl.

Durch die spezielle Ausbildung der erfindungsgemäßen Einrichtung wird eine separate Befüllbarkeit von in sich abgeschlossenen Räumen, der Kammern und/oder Kanälen, mit gasförmigen und/oder flüssigen Medien ermöglicht. Je nach Bedarf kann entweder eines dieser Medien alleine oder zwei dieser Medien in vermischter Form oder aber auch mögliche Zwischenstufen derselben an den zu behandelnden Körper abgegeben werden. Die zumindest zwei zwischen den Schichten angeordneten Kammern/Kanäle ermöglichen auch, an lokal unterschiedlichen Körperstellen, je nach Lage/Position der Auflage bzw. der Kammern und/oder Kanäle am Körper, eine Abgabe durchzuführen. Der Körper bzw. lokal verschiedene Körperstellen kann/können mit voneinander unterschiedlichen Medien gleichzeitig oder sogar zeitversetzt versorgt werden. Diese Abgabe, unter welcher selbstverständlich auch die Druckbeaufschlagung der Auflage an den Körper verstanden wird, wird zusätzlich durch die Steuer- bzw. Regeleinrichtung kontrolliert. Die Steuer- bzw. Regeleinrichtung umfasst nicht nur eine Vorrichtung mittels welcher eine Steuerung bzw. Regelung der in den Kammern und/oder Kanälen befindlichen Medien, wie beispielsweise Durchflussmenge oder Temperatur, ermöglicht wird, sondern auch geeignete Vorrichtungen bekannter und handelsüblicher Art, welche solche Medien zur Verfügung stellen, aufbereiten, mischen, messen, etc., und in einer dem Körper ausgerichteten Form den Kammern und/oder den Kanälen zugeführt werden können. So sind im Wesentlichen u.a. Behältnisse, wie Druckbehältnisse für jeweilige gasförmige bzw. flüssige Medien, Mischkammern zur Einstellung verschiedener Parameter der flüssigen und/oder gasförmigen Medien, wie Temperatur, Feuchtigkeitsgrad, Zusammensetzung der Medien, Mischungsverhältnis, Ionisationsgrad, etc., Pumpen, Rückstrombehältnisse mit Filtereinrichtungen zur Wiederaufbereitung der in den Kammern/Kanälen befindlichen Medien, Gasentfeuchtungsbehälter zur Wiederaufbereitung von wiederverwendbaren Gasen, Düsenpumpen, Kompressoren, Gebläse, Wärme- bzw. Kältetauscher, Ultraschallbefeuchter oder Bubbler, Düsen, Sensoren oder Messeinrichtungen, wie Hygrometer, Ionisationsmesser, Thermostaten und dergl., diverse Anschlüsse, Ventile, und dafür vorgesehene Zufuhr- bzw. Abfuhrleitungen, vorgesehen. Die Behältnisse bzw. Kammern mit ihren entsprechenden Zusatzeinrichtungen, wie Leitungen, sind vorzugsweise autonom, so dass in jedem Behältnis das gewünschte Medium separat eingesetzt werden kann und z.B. ungewünschte Reaktionsabläufe oder Interaktionen zwischen einzelnen Medien verhindert werden.

So ist es nun vorstellbar, dass Vorgänge in den Steuer- bzw. Regeleinrichtungen auch wie folgt ablaufen können: von einem. Versorgungsbehältnis, welches mit Flüssigkeit, wie beispielsweise destilliertes Wasser, gefüllt ist, wird eine bestimmte Menge zu einer Mischkammer geführt, um dort mit beispielsweise Gasen, wie O₂, CO₂, O₃ oder Gasgemischen, Duftstoffen oder Salzen versetzt zu werden. Während einer Weiterleitung dieses Mediengemisches wird dieses beispielsweise erwärmt, gekühlt oder anderwertig behandelt bzw. aufbereitet, und letztendlich zu den Kammern und/oder Kanälen transportiert um an den Körpern abgegeben zu werden. Selbstverständlich sind auch Vorgänge möglich, welche eine Durchleitung bzw. Zirkulation und anschließend entsprechende Umwälzung oder Wiederaufbereitung der Medien, wie z.B. Filterung oder andere Reinigung der Stoffe, zur Rückführung in die Versorgungsbehältnisse oder in die Kammern/Kanäle vorsehen.

Für solche Vorgänge, und v.a. für kombinierte Vorgänge dieser Art der gasförmigen und/oder flüssigen Medien müssen geeignete, wie auch bereits erwähnte, herkömmliche, bewährte Vorrichtungen in einfacher und/oder kombinierter Form vorhanden sein. In jedem Fall muss sichergestellt sein, dass sämtliche Vorgänge für eine Abgabe der chemisch-/physikalischen Parameter an den Körper keinerlei ungewünschte Abläufe hervorrufen können, so dass eine sichere, einwandfreie Handhabung der erfindungsgemäßen Einrichtung gestattet wird.

Eine Überwachung und Änderung der zur Abgabe an den Körper vorgesehenen, in den Kammern und/oder Kanälen befindlichen Medien kann gemäß der Erfindung zusätzlich vom zu behandelnden Körper selbst mittels der Biosensoren beeinflusst werden. Diese dienen, wie bereits erwähnt, der Erfassung von körperspezifischen Parametern, wie beispielsweise Hauttemperatur und -widerstand, EKG, EEG oder dergl., welche zur Steuer- bzw. Regeleinrichtung weitergeleitet werden. Derartige Sensoren stellen geeignete Messeinrichtungen dar, wie z.B. Thermometer, spezifische Konzentrationsmesser, elektrische Ableitungen etc., welche verschiedene Temperaturen, wie z.B. Kerntemperaturen, Periphertemperaturen, verschiedene Oberflächentemperaturen usw., elektrische Widerstände, Vitalwerte von Organismen, wie beispielsweise EEG, EKG, oder dergl., vom Körper ableiten. Die Steuer- bzw. Regeleinrichtung kann in Abhängigkeit der körperspezifischen Messwerte entsprechende Änderungen der Parameter, wie Temperatur, Druck, Durchflussmenge, Konzentration, Zusammensetzung oder dergl., der gasförmigen und/oder flüssigen Medien vornehmen. Dazu dient ein mit der Steuer- bzw. Regeleinrichtung und mit den Biosensoren verbundener Rechner, welcher die von den Biosensoren erhaltenen Messwerte verarbeitet und in geeigneter Form an die Steuer- bzw. Regeleinrichtung weiterleitet. Eine Verarbeitung der Messwerte im Rechner ist beispielsweise derart denkbar, dass vorzugsweise Minimal- und Maximalwerte oder gesamte Ablaufraster wie beispielsweise eines Temperaturverlaufes/-kurve in Computerprogrammen im Rechner gespeichert vorhanden sind. Ebenso ist es denkbar, dass diese Computerprogramme einfache oder aber auch komplexere Abläufe der gasförmigen bzw. flüssigen Medien und damit verbundenen Vorgänge erlauben, z.B. einen Druckverlauf der den Kammern/Kanälen zugeführten Gasen, um dadurch einen innerhalb eines vorbestimmten Rahmens definierten variierenden Anpressdruck der Auflage auf den Körper zu erzeugen, oder etwa eine bestimmte Dosierung einer Substanz in eine an den Körper abzugebende Flüssigkeit, oder aber auch parallele Vorgänge, wie stetig bis zu einem Maximalwert ansteigenden Druck der Gasbefüllung mit gleichzeitiger Abgabe von mit Wirkstoffen versehenen Dämpfen in bestimmter Konzentration, oder dergl. Diese im Rechner gespeicherten Informationen bzw. Abläufe werden an die entsprechenden Vorrichtungen der Steuer- bzw. Regeleinrichtung weitergegeben, welche die angesprochenen Vorgänge ausführt. In einer einfachen Ausführungsform werden die im Rechner gespeicherten Werte mit den von den Biosensoren aufgenommenen Messwerten des Körpers verglichen und ein daran angeglichener Wert, wie eine bestimmte Temperatur, wird an die Steuer- bzw. Regeleinrichtung weitergegeben, so dass diese entsprechende Änderungen oder Abweichungen der Parameter der flüssigen und/oder gasförmigen Medien, wie beispielsweise eine Temperaturänderung, vorgenommen wird. Somit wird ein kontrolliertes Wechselspiel bzw. eine gegenseitige Beeinflussung zwischen dem Körper und der Abgabe der gasförmigen und/oder flüssigen Medien durch die erfindungsgemäße Einrichtung erhalten.

Es soll an dieser Stelle noch erwähnt sein, dass zur Speisung/Befüllung bzw. zum Ablassen der Medien in bzw. aus den Kammern und/oder der Kanäle Leitungen vorzugsweise an seitlichen bzw. randlichen Ein- bzw. Auslässen der Auflage angeordnet sind, so dass der mit der Auflage in Kontakt befindliche Körper während einer Betriebsstellung der erfindungsgemäßen Einrichtung nicht durch Leitungen und/oder Ein- bzw. Auslässen irritiert wird.

In Zusammenhang mit der direkten Abgabe der chemisch-/physikalischen Parameter, wie beispielsweise Dampf, ist es auch besonders vorteilhaft, wenn wenigstens eine Schicht der Auflage, vorzugsweise die dem Körperteil zugewandte Schicht, durchlässig oder halbdurchlässig ist. Bevorzugterweise ist diese Schicht einseitig durchlässig, so dass die Medien nur in eine Richtung, nämlich nach außen gelangen können, aber keine Stoffe in die Kammern bzw. Kanäle eindringen können. Beim Befüllen bzw. Durchströmen der Kammern oder Kanäle können diese gasförmigen bzw. flüssigen Medien über die durch- bzw. halbdurchlässige Schicht hindurch ohne Behinderung auf die entsprechenden Körperstellen ohne Hinderungen gelangen. Ein direkter Kontakt der flüssigen und/oder gasförmigen Medien in Form von beispielsweise heilenden/wohltuenden Substanzen als Inhaltsstoffe der Medien mit der Haut des Körpers steigert bekannterweise den Wirkungsgrad und das Wohlbefinden eines damit in Kontakt tretenden Patienten. Die Durchlässigkeit bzw. Halbdurchlässigkeit kann durch verschiedene Öffnungen oder Porentypen gestaltet sein, wie beispielsweise einfache Poren, wie Rundporen, Einschnitte, sich überkreuzende Einschnitte, Ventile, einfaches durchlässiges bzw. halbdurchlässiges Gewebe oder dergl. Selbstverständlich ist eine Kombination derartiger Durchgänge, welche ein ungehindertes In-Kontakt-Treten der Medien mit der Körperoberfläche sicherstellt, ebenfalls möglich.

Für eine optimale Anwendung der erfindungsgemäßen Einrichtung ist es besonders günstig, wenn wenigstens eine Schicht der Auflage, vorzugsweise die dem Körperteil abgewandte Schicht, für die in den Kammern und/oder Kanälen befindlichen Stoffe undurchlässig ist. Dadurch wird erreicht, dass ein ungewollter Austritt der in den Kammern und/oder Kanälen befindlichen Medien aus der dem Körper oder Teilen davon entferntesten Schicht vermieden wird und die Abgabe der Stoffe gezielt über die dem Körper zugewandte Schicht erfolgt. Ebenso wird eine Leitung der Medien in den Kammern und/oder Kanälen leichter regulierbar, da ein Austritt der Stoffe nur in eine Richtung; nämlich über die Öffnungen der an der dem Körper zugewandten Seite der Kammern/Kanäle, erlaubt wird.

Neben der Abgabe der chemisch-/physikalischen Parameter über die Kammern nahe der dem Körperteil zugewandten Schicht ist es weiters von Vorteil, wenn wenigstens eine der Schichten zusätzlich zumindest einen mit gasförmigen und/oder flüssigen Medien befüllbaren Kanal aufweist. Bevorzugterweise sind diese Kanäle ebenfalls an der dem Körper zugewandten Schicht angeordnet und sind wie in einer oben angeführten Ausführungsform durchlässig bzw. halbdurchlässig, so dass zusätzlich gleiche oder verschiedene Stoffe über derartige Kanäle direkt an die Körperoberfläche gezielt abgegeben werden können. In einer solchen Weise wird eine regulierbare Abgabe von flüssigen bzw. gasförmigen Medien erhöht. Selbstverständlich kann die Beschaffenheit der Kanalummantelungen auch undurchlässig gestaltet sein, so dass beispielsweise nur eine Druckabgabe (Druckbeaufschlagung) durch die Kanäle stattfindet, wobei aber auch Kombinationen der Kanalummantelungen möglich sind. Es ist auch vorstellbar, dass diese Kanäle nicht einer reinen Abgabe dienen, sondern zur Ableitung bzw. Abtransport von Rückständen oder Überständen von gasförmigen und/oder flüssigen Medien verwendet werden. Solche Rückstände oder Überstände können sich bei einer Anwendung der erfindungsgemäßen Einrichtung leicht zwischen der Auflage und dem Körper ansammeln, so dass eine ungehinderte und gezielte Aufnahme von Stoffen über die Haut des Körpers nicht mehr sicher garantiert ist. In einem solchen Fall ist es besonders vorteilhaft, wenn derartige Rückstände über die Kanäle beispielsweise durch leichte Ansaugkraft abtransportiert werden.

Für eine flexiblere Ausgestaltung der erfindungsgemäßen Einrichtung sind die Kanäle an der dem Körperteil zugewandten Schicht vorzugsweise lösbar angebracht, so dass sie beliebig, je nach Erfordernis der zu behandelnden Körperstelle variabel angeordnet werden können. Ein derartiges Anbringen kann durch beispielsweise Klettverschlüssen, Druckknöpfen oder sonstigen Befestigungsmittel vorgesehen sein, welche eine lösbare Verbindung an einer Schicht der Auflage schaffen.

Eine weitere vorteilhafte Ausführungsform der erfindungsgemäßen Einrichtung ist dadurch gekennzeichnet, dass die Kammern der Auflage jeweils in weitere miteinander kommunizierende Unterkammern unterteilbar sind. Durch eine derartige Unterteilung ist es möglich, dass mehrere Kammern bzw. Unterkammern vorgesehen sind, wobei jede dieser Unterteilungen gasförmige und/oder flüssige Medien mit unterschiedlicher Zusammensetzung enthalten können. So ist beispielsweise vorstellbar, dass die Kammern bzw. Unterkammern übereinander angeordnet sind, wobei zwischen den Kammern bzw. Unterkammern eine weitere Schicht zur Trennung der Kammern verläuft und diese vorzugsweise undurchlässig ist. Bei einer solchen Anordnung ist es besonders effektiv, wenn wenigstens eine dem Körper zugewandte Kammer rein zur Abgabe der chemisch-/physikalischen Parameter dient, und wenigstens eine dem Körper abgewandte Kammer der Formgebung. Eine Kommunikation zwischen den Unterteilungen kann durch unterschiedliche Öffnungen, wie beispielsweise Ventile oder verschiedene Portentypen (wie oben erwähnt), gestaltet sein, welche einen mehr oder weniger leichten Durchgang der in den Unterteilungen/Kammern befindlichen Medien erlaubt.

Weiters ist es von Vorteil, wenn die Kanäle ineinander verlaufend angeordnet sind. Dies schafft die Möglichkeit, dass in einem Kanal ein innerer Kanal vorgesehen sein kann, wobei diese Kanäle beispielsweise koaxial zueinander angeordnet sein können. Die ineinander verlaufenden Kanäle können eine durchlässige bzw. halbdurchlässige bzw. undurchlässige Ummantelung aufweisen, je nach Befüllung durch gasförmige und/oder flüssige Medien, wobei darauf geachtet werden sollte, dass keine ungewollte Interaktion zwischen Gas und Flüssigkeit stattfinden kann. Hingegen ist es möglich, dass beispielsweise der innere Kanal mit Gasen, wie O₂, CO₂, O₃, getrennt befüllt wird und durch seine durchlässige Ummantelung das genannte Gas in den umliegenden Kanal, welcher beispielsweise mit Flüssigkeit gefüllt ist, dringt, so dass eine Begasung der Flüssigkeit während einer Anwendung der erfindungsgemäßen Einrichtung erfolgen kann. Eine Anwendung dieser Weise kann auch beispielsweise derart durchgeführt werden, dass zwei oder mehrere innere Kanäle nebeneinander angeordnet sind und gasförmige und/oder flüssige Medien unterschiedlicher Zusammensetzung beinhalten und das im umliegenden Kanal befindliche gasförmige und/oder flüssige Medium entsprechend versorgen, um dieses dann anschließend direkt an den zu behandelnden Körper abzugeben. Eine nebeneinander angeordnete Ausgestaltung der Kanäle bzw. inneren Kanäle ist auch besonders zielführend, da die Medien unterschiedlicher Zusammensetzung einzeln und insbesondere dosiert an die Hautoberfläche des Körpers abgegeben werden können.

Für eine optimale Kontrolle und präzise Anpassung an die Bedürfnisse des mit der Auflage umhüllten bzw. bedeckten Körpers oder Patienten ist es weiters von Vorteil, wenn die Steuer- bzw. Regeleinrichtung mit Ventilen in Leitungen für die gasförmigen und/oder flüssigen Medien zur Regelung der Durchflussmenge der gasförmigen und/oder flüssigen Medien verbunden ist. Dadurch wird sichergestellt, dass eine gezielte Abgabe der Medien an den Körper oder Teilen davon erreicht wird, nämlich dadurch, dass durch Öffnung der Ventile in den Leitungen die entsprechende Durchflussmenge erhöht wird und somit eine erhöhte Menge bzw. erhöhte Konzentration von gasförmigen und/oder flüssigen Medien direkt an den Körper oder Teilen davon abgegeben werden kann. Somit ist es ebenfalls möglich, unterschiedliche Stellen des Körpers mit verschiedenen Medien bzw. verschiedener Zusammensetzung zu versorgen oder beispielsweise auch mit unterschiedlichem Druck zu beaufschlagen.

An dieser Stelle sei anzumerken, dass bei beispielsweise Funktionsausfall der Biosensoren oder des Rechners die erfindungsgemäße Einrichtung, insbesondere die Steuer- bzw. Regeleinrichtung, manuell steuer- bzw. regelbar ist, so dass ungewünschte Abläufe hintangehalten werden können.

Zudem ist noch zu erwähnen, dass die Auflage vorteilhafterweise aus biegsamem Material, wie beispielsweise gerecktem Polytetrafluorethylen oder Polyvinylchlorid, hergestellt sind. Ein derartiges Material stellt eine optimale Anpassung der Auflage an den mit der Auflage angelegten Körper oder Teilen davon sicher. Weiters ist ein solches Material hitzebeständig und zeigt auch gegenüber ölige Substanzen oder Salzen abweisende Eigenschaften. Zudem ermöglicht es eine mehrmalige Verwendung der Auflage, da dieses Material durch seine Langlebigkeit und Abwaschbarkeit ausgezeichnet ist.

Vorzugsweise sind die Auflage und, falls gewünscht, auch die Biosensoren der erfindungsgemäße Einrichtung in einer den Körper oder Teilen davon zumindest teilweise umschliessenden, formstabilen Ummantelung angeordnet. Diese Ausführungsform ist beispielsweise für die Einrichtung von Brüchen besonders geeignet, durch Druckbeaufschlagung einzelner Kammern kann unter Abstützung an der Ummantelung auf bestimmte Stellen des Körpers oder Teilen davon gezielt und dosiert Druck ausgeübt werden, wobei die Auswirkung des Druckes z.B. über Röntgenkontrolle beobachtet werden kann. Abgesehen davon kann auch durch periodisches Druckbeaufschlagen bzw. Entlasten bestimmter Zellen ein Massageeffekt bewirkt werden, der z.B. mittels Einleiten verschiedener Stoffe, Gase, Cremen, Heilwässer, Aromastoffe, Duftöle etc. durch die Kanäle auch verstärkt werden kann. Die Cremen können auch vor Behandlung auf den Körper aufgetragen werden, wobei der oben beschriebene Massageeffekt z.B. durch Einleiten von Gasen (Ozon, Kohlendioxyd, Stickstoff udgl.) modifizierbar ist. Auch können Heilbäder unter Verwendung minimaler Mengen an Heilwässer durchgeführt werden. Auflage und Ummantelung können auch in Form von einfachen Kleidungsstücken (Overalls) ausgebildet sein, wobei durch die Druckbeaufschlagung z.B. bei der Unterwassertherapie Auftrieb oder Abtrieb'erzeugt werden kann, wodurch der Körper bzw. Körperteil vorsichtig bewegt werden kann. Beispielsweise bei der Rehabilitation von Lähmungen kann dadurch erstmals eine einfache, gezielte Bewegungstherapie samt Rückmeldung über die Biosensoren vorgesehen werden. Die Verbindungsleitungen zwischen Auflage, Biosensoren und Steuer/Regeleinrichtung kann dabei derart ausgebildet sein, daß sie leicht lösbar sind bzw. sicher unterbrochen werden können.

Weiters ist es auch vorstellbar, dass die Auflage gemäß der Erfindung als Auskleidung in einer Wanne angebracht, vorteilhafterweise gespannt, werden kann. Dafür kann auch gegebenenfalls eine Spannvorrichtung vorgesehen sein, welche beispielsweise auch ein Heben bzw. Senken der Auflage in der Wanne erlaubt, so dass ein zu behandelnder Körper auf der Auflage zu liegen kommt und in die Wanne herabgesenkt wird. Bei einer Anwendung der erfindungsgemäßen Einrichtung in dieser Art ist es auch denkbar, dass die Wanne selbst mit gasförmigen und/oder flüssigen Medien befüllbar ist und diese Medien selbst einen Anpressdruck zur Formgebung der Auflage an den Körper ausüben. Demgemäß kann die Auflage nur rein zur Abgabe, beispielsweise von wohltuenden Dämpfen, eingesetzt werden. Eine Anwendung/Einsatz der erfindungsgemäßen Einrichtung gemeinsam mit einer derartigen Wanne kann noch zusätzlich wirkungsvoll erhöht werden, wenn beispielsweise andere Heil- oder wohltuende Mittel, wie Heu, Moorpackungen oder dergl., zwischen Auflage und Körper eingelegt werden. Dabei sollte nur darauf geachtet werden, dass die Öffnungen der Kammern bzw. Kanäle an der dem Körper zugewandten Seite nicht verstopft oder verlegt werden, so dass ein ungehinderter Zugang bzw. Durchgang der in den Kammern und/oder Kanälen befindlichen Medien noch gestattet wird.

Die Erfindung wird nachstehend anhand von in der Zeichnung veranschaulichten vorteilhaften Ausführungsbeispielen, auf die sie jedoch nicht beschränkt sein soll, näher erläutert.

Im Einzelnen zeigen:
Fig. 1 einen Querschnitt der erfindungsgemäßen Auflage mit zwei Kammern und einer durchlässigen bzw. halbdurchlässigen, dem Körperteil zugewandten Schicht;
Fig. 2 einen Querschnitt durch die erfindungsgemäße Auflage mit einer Unterteilung der Kammern in vier Kammern und mit Kanälen an der dem Körperteil zugewandten Schicht;
Fig. 3a bis 3e Querschnitte durch verschieden ausgestaltete Kanäle.

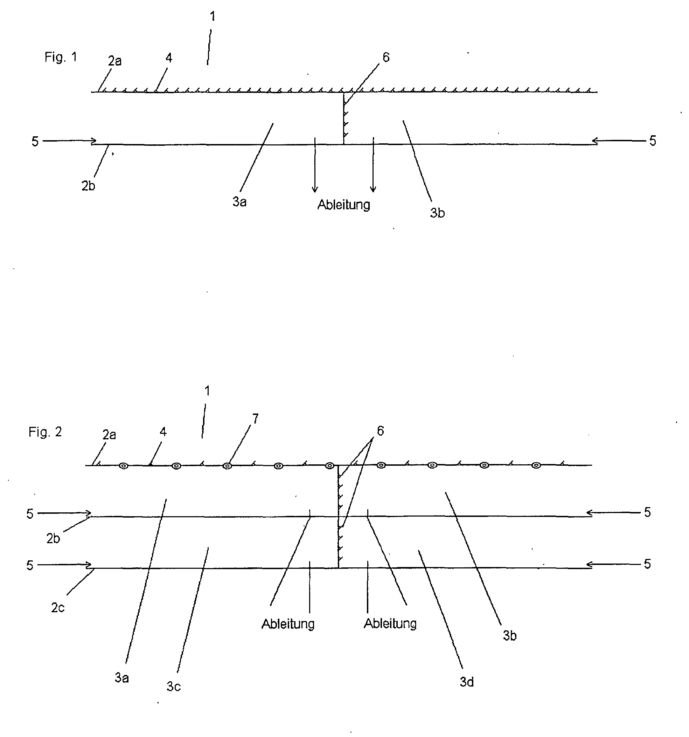

Die Auflage 1 der erfindungsgemäßen Einrichtung gemäß Fig. 1 enthält als wesentliche Bestandteile zwei Schichten 2a, 2b, wobei der Raum zwischen den Schichten 2a, 2b zwei Kammern 3a, 3b aufweist, welche jeweils seitlich bzw. im Randbereich mit Leitungen 5 ausgestattet sind. Die dem Körper zugewandte Schicht 2a (hier die obere Schicht) ist durchlässig bzw. halbdurchlässig (Öffnungen 4) und die dem Körper abgewandte Schicht 2b (hier die untere Schicht) undurchlässig. In diesem einfachen Ausführungsbeispiel gemäß der vorliegenden Erfindung wird die Auflage 1 an einen Körper oder Teilen davon angelegt, so dass die mit den Öffnungen 4 versehene Schicht 2a an der Körperoberfläche zu liegen kommt. Die dem Körper abgewandte Schicht 2b dient in diesem einfachen Ausführungsbeispiel als Formgebungsschicht und weist isolierende Eigenschaften auf. Sodann wird über die Leitungen 5 seitlich gasförmiges und/oder flüssiges Medium, wie beispielsweise mit Duftstoffen versetzte Flüssigkeit, in die Kammer 3a gefüllt, wobei die Befüllung mittels der Steuer- bzw. Regeleinrichtung (hier nicht gezeigt) durchgeführt und kontrolliert wird. Diese beiden Kammern 3a, 3b können ihrerseits durch ein oder mehrere Öffnungen 6 kommunizieren, so dass das in die Kammer 3a einströmende Medium, auch in die zweite Kammer 3b dringen kann. In der Kammer 3a kann das Medium mittels beispielsweise Heizdrähten (hier nicht gezeigt) auf seinen Siedepunkt erwärmt werden, so dass es über die Öffnung 6 in dampfförmigen Zustand auch in die Kammer 3b gelangt, um in beiden Kammern über die Öffnungen 4 an die Körperoberfläche (der Haut) abgegeben zu werden. Nach Abschluss der Anwendung wird das Medium über die seitlich gelegene Leitung 5 der Kammer 3b abtransportiert. Eine Zu- bzw. Ableitung wird in der Regel mittels Pumpen der Steuer- bzw. Regeleinrichtung (hier nicht abgebildet) erreicht. Je nach Anwendungsanforderung können die Kammern 3a und 3b auch über die seitlichen Leitungen 5 einzeln gefüllt bzw. angeströmt werden, wobei dann die Öffnung 6, die die Kammern 3a, 3b verbindet, geschlossen bleibt. In diesem Fall kann die zu behandelnde Körperstelle mit zwei Medien unterschiedlicher Zusammensetzung versorgt werden. Bei Abschluss der Anwendung übernehmen die Leitungen 5 auch einen Abtransport der Medien aus den jeweiligen Kammern 3a, 3b.

Wie bereits in der Beschreibungseinleitung erwähnt, dient die Steuer- bzw. Regeleinrichtung nicht nur einer Befüllung der Kammern 3a, 3b bzw. den Kanälen (siehe Fig. 2), sondern auch der Steuerung bzw. Regelung der gasförmigen und/oder flüssigen Medien (in Fig.1 nicht abgebildet). So ist die Steuer- und Regeleinrichtung u.a. mit einem Rechner ausgestattet, welcher z.B. ein Ablaufraster (Computerprogramm) eines speziellen Abgabeschemas gespeichert hält. Beispielsweise kann ein bestimmter Temperaturverlauf der den Kammern/Kanälen zugeführten Medien aufgerufen werden, um durch innerhalb des Ablaufrasters definierten minimalen und maximalen Temperaturangaben verlaufende Temperaturänderungen der Medien zu erzeugen. Diese Informationen werden an die für eine Temperaturänderung vorgesehenen Vorrichtungen der Steuer- bzw. Regeleinrichtung geleitet und dort umgesetzt, so dass die Medien entsprechend des vorbestimmten Temperaturverlaufes temperiert werden.

Bei einer Anwendung wird ein gespeichertes und aufgerufenes Abgabeschema der Medien mittels der Steuer- bzw. Regeleinrichtung umgesetzt und die Kammern/Kanäle demgemäß befüllt bzw. gesteuert bzw. geregelt (in Fig.1 nicht abgebildet). Die angelegten Biosensoren (in Fig. 1 nicht gezeigt) nehmen, je nach verwendeten Sensoren, verschiedene Parameter der entsprechenden Körperstellen auf, wobei erwähnt sein soll, dass eine Kombination aus unterschiedlichen Sensoren für verschiedene Parameter ebenfalls verwendet werden kann. Wenn z.B. die Biosensoren an einer bestimmten Körperstelle mit der sie verbunden sind, eine erhöhte Temperatur erfassen, wird dieser Wert an den Rechner der Steuer- bzw. Regeleinrichtung weitergeleitet, und mit einem innerhalb des aufgerufenen Ablaufrasters bereits gespeicherten Wert verglichen. Entspricht dieser nicht einem innerhalb des Rasters definierten Wert, so wird eine Korrektur vorgenommen und der in der Steuerund Regeleinrichtung dafür vorgesehene Temperaturregler (in Fig. 1 nicht gezeigt) ändert seine gemäß des Ablaufrasters voreingestellten Einstellungen und die Temperatur in dem in der'Kammer 3a oder 3b befindlichen Medium wird gemäß der erfassten Temperatur geändert, so dass die betroffene Körperstelle mit dem Temperaturgesenkten Medium versorgt wird, und es zu keiner lokalen Überhitzung der Haut an dieser Stelle kommt.

Bezüglich einer Druckbeaufschlagung bzw. Formgebung werden in der in Fig. 1 dargestellten Auflage 1 die Kammern 3a, 3b zur optimalen Anpassung der Auflage 1 an den damit angelegten Körper z.B. mit Gasen oder Flüssigkeiten gefüllt, wobei auch in diesem Ausführungsbeispiel gemäß im Rechner gespeicherte Druckangaben ausgeführt werden. Sobald von den Biosensoren eine zu starke Druckbelastung einer der Kammer 3a nächstgelegenen bestimmten Körperstelle erfasst wird, wird mittels des Rechners der Wert korrigiert und von der Steuer- bzw. Regeleinrichtung, mittels beispielsweise Ventile, die Durchflussmenge des Gases/der Flüssigkeit durch die Kammern 3a verringert, so dass die betroffene Körperstelle entlastet wird.

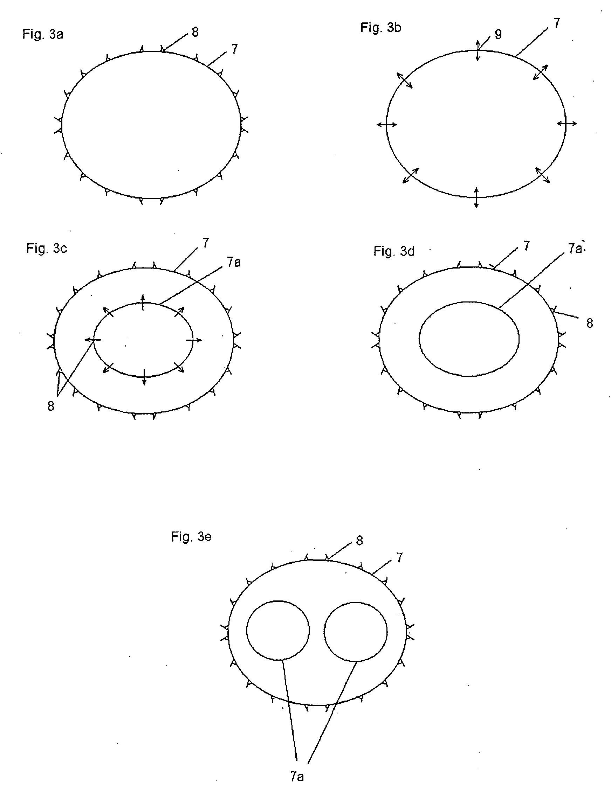

Fig. 2 stellt ein komplexeres Ausführungsbeispiel der erfindungsgemäßen Einrichtung dar, wobei der Raum zwischen den Schichten 2a, 2b, 2c der Auflage 1 in vier Kammern 3a, 3b, 3c, 3d unterteilt ist, welche übereinander und nebeneinander angeordnet sind. Die dem Körper abgewandteste Schicht 2c (hier unterste Schicht) ist undurchlässig dargestellt (durchgehende Linie), die Zwischenschicht 2b (hier mittlere Schicht) ist ebenfalls undurchlässig und die dem Körper zugewandte Schicht 2a (oberste Schicht) ist durchlässig bzw. halbdurchlässig dargestellt. Leitungen 5 (im Rand- bzw. Seitenbereich) versorgen die Kammern 3a, 3b, 3c, 3d mit flüssigen und/oder gasförmigen Medien, wobei diese, insbesondere die Kammern 3a, 3b, über Öffnungen 4 der Schicht 2a wieder verlassen können. Weiters zeigt Fig. 2 Kanäle 7 bzw. zwei ineinander verlaufende Kanäle 7, welche an der dem Körper zugewandten Schicht 2a angeordnet sind und in Fig. 3 detailliert gezeigt sind. Hier in Fig. 2 sind die Kanäle 7 in einem regelmäßigen Abstand zueinander angeordnet, wobei zwischen jedem Kanal 7 eine Öffnung 4 vorgesehen ist und die Kanäle 7 quer zur Durchströmungsrichtung der gasförmigen bzw. flüssigen Medien in den Kammern 3a, 3b, 3c, 3d verlaufen.

Beim Anlegen der erfindungsgemäßen Auflage 1 an einen zu behandelnden Körper oder Teilen davon (nicht gezeigt) dienen die in Fig. 2 dargestellten Kammern 3a und 3b zur regulierten Abgabe von flüssigen und/oder gasförmigen Medien, während die dem Körper abgewandten Kammern 3c, 3d zur Formgebung bzw. Druckbeaufschlagung vorgesehen sind. D.h., es wird in Kammern 3c, 3d mehr oder weniger gasförmiges bzw. flüssiges Medium gefüllt, so dass die Kammern 3a, 3b sicher und mit geregeltem Druck am Körper anliegen. Die Kammern 3a, 3b dienen in diesem Fall rein zur Abgabe der flüssigen bzw. gasförmigen Medien, welche in gleicher Weise erfolgt wie bereits bei den Erläuterungen der Fig. 1 ausgeführt, nämlich Befüllung der Kammern 3a, 3b, 3c, 3d gemäß eines im Rechner der Steuer- bzw. Regeleinrichtung gespeicherten und abgerufenen Ablaufschemas, Erfassung von Körperparametern mittels der Biosensoren und entsprechende Regulierung bzw. Kontrolle der Vorgänge der in den Kammern 3a, 3b, 3c, 3d befindlichen Medien mittels der Steuer- bzw. Regeleinrichtung. In diesem Fall kann eine Druckentlastung in kontrollierbarer Form, wie beispielsweise durch Einwegventile (nicht gezeigt), über die Öffnungen 4 oder über die seitlich bzw. randlich angeordneten Abflussleitungen erwirkt werden.

Die Kanäle 7 der Schicht 2a (Fig. 2) sind ebenfalls, wie die Kammern 3a, 3b, 3c, 3d über die Steuer- und Regeleinrichtung und damit ausgestatteten Rechner (nicht gezeigt) mit flüssigen und/oder gasförmigen Medien einzeln befüllbar und kontrollierbar.

Je nach Verwendung der Kanäle 7 als Abgabemittel oder rein als Druckbeaufschlagungsmittel, weisen die Kanäle 7 durchlässige bzw. halbdurchlässige Ummantelungen (siehe Fig. 3) auf. Durch ihre Anordnung an der Schicht 2a wird eine lokale Wirkung besonders zu behandelnder Körperstellen erhöht. Dabei wird je nach mittels der Biosensoren erfasster Werte beispielsweise die Konzentration der durch die Kanäle fließenden Medien erhöht und gesteuert bzw. geregelt nur an die betroffene Körperstelle abgegeben. Die Kanäle 7 können separat eingesetzt werden, so dass verschiedene Körperstellen, welche unterschiedliche Reaktionen zeigen, über die Biosensoren und entsprechend über die Steuer- bzw. Regeleinrichtung versorgt werden.

Die Kanäle 7 können auch als Abflussleitungen der von den Kammern 3a, 3b abgegebenen Medien verwendet werden, wobei sich ein Kreislauf nicht nur über die Zu- und Abfuhr in und aus den Kammern 3a, 3b, 3c, 3d ergibt, sondern auch eine zusätzliche Abfuhr der von den Kammern 3a, 3b abgegebenen Stoffe über die Kanäle 7 erfolgt.

Fig. 3 zeigt verschiedene Formen der Kanäle 7 und unterschiedlichen Ummantelungen, wobei im Einzelnen Fig. 3a einseitig durchlässige Öffnungen 8 für flüssige bzw. gasförmige Medien zeigt. Hier können zwar Flüssigkeiten oder Gase nach außen gelangen, jedoch nicht wieder in den Kanal 7 eindringen. Fig. 3b weist beidseitig durchlässige Öffnungen 9 für gasförmige bzw. flüssige Medien auf, welche besonders für einen Abtransport der überschüssigen Medien aus der Umgebung des Kanals 7 geeignet sind. In Fig. 3c sind zwei ineinander verlaufende Kanäle 7, 7a, welche koaxial zueinander angeordnet sind, mit jeweils einseitig durchlässigen Öffnungen 8 für Gase und Flüssigkeiten dargestellt. Hier kann der innere Kanal 7a ein gasförmiges Medium führen, während der umliegende Kanal 7 mit einer Flüssigkeit durchströmt wird. Die Öffnungen 8 des inneren Kanals 7a erlauben durch ihre einseitige Durchlässigkeit eine Begasung der im umliegenden Kanal 7 befindlichen Flüssigkeit mit dem Gas des inneren Kanals 7a, wobei allerdings eine negative Beeinflussung des Gases durch die Flüssigkeit nicht möglich ist. Dieses Flüssigkeitsgasgemisch kann weiter über die im umliegenden Kanal 7 befindlichen Öffnungen 8 an den Körper abgegeben werden.

Weiters sind in Fig. 3d die Kanäle 7 und 7a wieder koaxial zueinander angeordnet, wobei der innere Kanal 7a ein gasförmiges Medium und der umliegende Kanal 7 ein flüssiges Medium enthält. Der mit Gas befüllte innere Kanal 7a kann zusätzlich zur Druckbeaufschlagung eingesetzt werden, wobei gleichzeitig eine Abgabe eines flüssigen Mediums durch den mit einseitigen Öffnungen 8 versehenen umliegenden Kanal 7 erfolgen kann.

Wie in Fig. 3e gezeigt, können z.B. zwei oder mehrere innere Kanäle 7a nebeneinander angeordnet sein, die durch einen umgebenden Kanal 7 geführt werden. Dabei können die Ummantelungen der entsprechenden inneren Kanäle 7a bzw. dem umgebenden Kanal 7 mit Kombinationen bezüglich ihrer Durchlässigkeit ausgestalteter Öffnungen versehen sein, so dass als Resultat der Interaktion zwischen den einzelnen Medien gezielt gewünschte Mediengemische gebildet werden können und diese an die entsprechende Körperstelle weitergeleitet werden können. Im vorliegenden Fall weist der Kanal 7 einseitig Durchlässige Öffnungen 8 auf, die inneren Kanäle 7a sind wie in Fig. 3d ausgebildet.

Zusammenfassend kann gesagt werden, dass durch das Zusammenspiel der Biosensoren mit den verbundenen Steuer- und Regeleinrichtung verschiedene Medien unterschiedlicher Komposition, wie beispielsweise unterschiedliche Sättigung, Mischungsverhältnis, Konzentration, Druck, Temperatur, Aggregatzustand, etc. effektiv und einfach an einen optimal an den zu behandelnden Körper in Abhängigkeit seiner Reaktion angepasst und abgegeben werden können und v.a. zielgerecht und ohne jegliche Hinderungen dorthin geleitet werden können, wo eine gewünschte Wirkung erzielt werden soll.

## Patentansprüche

1. Einrichtung zur Abgabe chemisch-/physikalischer Parameter und zum Anlegen an Körper oder Teilen davon, wobei die Einrichtung eine Auflage (1) mit wenigstens zwei Schichten aufweist und der zwischen den Schichten bestehende Raum in mindestens zwei Kammern und/oder Kanäle unterteilt ist, welche mit gasförmigen und/oder flüssigen Medien zur Abgabe der chemisch-/physikalischen Parameter befüllbar sind, und die Einrichtung weiters zumindest eine Steuer- bzw. Regeleinrichtung zur Steuerung bzw. Regelung von Parametern, wie beispielsweise Durchflussmenge, Temperatur, Druck oder dergleichen, der in dem Raum befindlichen Medien aufweist, die mit der Auflage verbunden ist, wobei Biosensoren zur Erfassung von Körperparametern, wie beispielsweise Temperatur, EKG oder dergleichen, mit der Steuerund Regeleinrichtung verbunden sind, **dadurch gekennzeichnet, dass** die Kammern (3a, 3b, 3c, 3d) und/oder Kanäle unabhängig voneinander mit den Medien befüllbar sind, und dass die Medien in den jeweiligen Kammern (3a, 3b, 3c, 3d) und/oder Kanälen über die Steuer- bzw. Regeleinrichtung in Abhängigkeit der mittels der Biosensoren erfassten Körperparameter steuer- bzw. regelbar sind.

2. Einrichtung nach Anspruch 1, **dadurch gekennzeichnet, dass** zwischen den Kammern (3a, 3b, 3c, 3d) verschließbare Öffnungen (6) vorgesehen sind.

3. Einrichtung nach Anspruch 1 oder 2, **dadurch gekennzeichnet, dass** die Kammern (3a, 3b, 3c, 3d) und/oder Kanäle nebeneinander und/oder untereinander angeordnet sind.

4. Einrichtung nach einem der Ansprüche 1 bis 3, **dadurch gekennzeichnet, dass** wenigstens eine Schicht (2a, 2b, 2c) der Auflage (1), vorzugsweise die dem Körperteil zugewandte Schicht (2a), zur Abgabe der gasförmigen und/oder flüssigen Medien an den Körperteil durchlässig oder halbdurchlässig ist.

5. Einrichtung nach Anspruch 4, **dadurch gekennzeichnet, dass** die Schicht (2a) Öffnungen (4), Poren, Ventile, halbdurchlässiges Gewebe oder dergleichen aufweist.

6. Einrichtung nach einem der Ansprüche 1 bis 5, **dadurch gekennzeichnet, dass** wenigstens eine Schicht (2a, 2b, 2c) der Auflage (1), vorzugsweise die dem Körperteil abgewandte Schicht (2b, 2c), für die in den Kammern (3a, 3b, 3c, 3d) und/oder Kanälen befindlichen Medien undurchlässig ist.

7. Einrichtung nach einem der Ansprüche 1 bis 6, **dadurch gekennzeichnet, dass** wenigstens eine der Schichten (2a, 2b, 2c) zumindest einen mit gasförmigen und/oder flüssigen Medien befüllbaren Kanal (7) aufweist.

8. Einrichtung nach Anspruch 7, **dadurch gekennzeichnet, dass** zumindest ein Kanal (7) für die Abgabe von gasförmigen und/oder flüssigen Medien durchlässig oder halbdurchlässig ist.

9. Einrichtung nach Anspruch 8, **dadurch gekennzeichnet, dass** zumindest ein Kanal (7, 7a) Öffnungen (8, 9), beispielsweise einseitig durchlässige Öffnungen (8) oder beidseitig durchlässige Öffnungen (9), aufweisen.

10. Einrichtung nach einem der Ansprüche 7 bis 9, **dadurch gekennzeichnet, dass** zumindest ein Kanal (7, 7a) für die gasförmigen und/oder flüssigen Medien undurchlässig ist.

11. Einrichtung nach einem der Ansprüche 7 bis 10, **dadurch gekennzeichnet, dass** die Kanäle (7) an der dem Körperteil zugewandten Schicht (2a) der Auflage angeordnet sind.

12. Einrichtung nach einem der Ansprüche 7 bis 11, **dadurch gekennzeichnet, dass** die Kanäle (7) lösbar an der Schicht (2) der Auflage (1) angebracht sind.

13. Einrichtung nach einem der Ansprüche 7 bis 12, **dadurch gekennzeichnet, dass** die Kanäle (7, 7a) ineinander verlaufend angeordnet sind.

14. Einrichtung nach einem der Ansprüche 1 bis 13, **dadurch gekennzeichnet, dass** die Kammer bzw. die Kammern (3a, 3b, 3c, 3d) jeweils in weitere miteinander kommunizierende Unterkammern unterteilt sind.

15. Einrichtung nach einem der Ansprüche 1 bis 14, **dadurch gekennzeichnet, dass** die Steuer- bzw. Regeleinrichtung mit Ventilen in Leitungen (5) für die gasförmigen und/oder flüssigen Medien zur Regelung der Durchflussmenge der gasförmigen und/oder flüssigen Medien verbunden ist.

16. Einrichtung nach einem der Ansprüche 1 bis 15, **dadurch gekennzeichnet, dass** die Schichten (2a, 2b, 2c) der Auflage (1) aus biegsamem Material, wie beispielsweise gerecktem Polytetrafluorethylen oder Polyvinylchlorid hergestellt sind.

17. Einrichtung nach einem der Ansprüche 1 bis 16, **dadurch gekennzeichnet, dass** die Auflage (1) und, falls gewünscht, auch die Biosensoren der erfindungsgemäßen Einrichtung in einer den Körper oder Teilen davon zumindest teilweise umschließenden, formstabilen Ummantelung angeordnet sind.

## Claims

1. A device for releasing chemical/physical parameters and for applying to bodies or parts thereof, wherein the device comprises an applicator (1) with at least two layers, and the space between the layers is subdivided into at least two chambers and/or channels which are fillable with gaseous and/or liquid media for releasing the chemical/physical parameters, and the device further comprises at least one controlling and regulating device for controlling, or regulating, respectively, parameters, such as, e.g., the flowthrough volume, temperature, pressure or the like, of the media present in the space, which is connected to the applicator, wherein biosensors for detecting body parameters, such as, e.g., temperature, EEG, or the like, are connected to the control- and regulating device, **characterised in that** the chambers (3a, 3b, 3c, 3d) and/or channels are independently fillable with the media, and **in that** the media in the respective chambers (3a, 3b, 3c, 3d) and/or channels can be controlled or regulated, respectively, by the control or regulating device in dependence on the body parameters detected by the biosensors.

2. A device according to claim 1, **characterised in that** closable openings (6) are provided between the chambers (3a, 3b, 3c, 3d).

3. A device according to claim 1 or 2, **characterised in that** the chambers (3a, 3b, 3c, 3d) and/or channels are arranged next to and/or under one another.

4. A device according to any one of claims 1 to 3, **characterised in that** at least one layer (2a, 2b, 2c) of the applicator (1), preferably the layer (2a) that faces the body part, is permeable or semipermeable for releasing the gaseous and/or liquid media onto the body part.

5. A device according to claim 4, **characterised in that** the layer (2a) has openings (4), pores, valves, semipermeable woven fabrics or the like.

6. A device according to any one of claims 1 to 5, **characterised in that** at least one layer (2a, 2b, 2c) of the applicator (1), preferably the layer (2b, 2c) that faces away from the body part, is impermeable to the media present in the chambers (3a, 3b, 3c, 3d) and/or channels.

7. A device according to any one of claims 1 to 6, **characterised in that** at least one of the layers (2a, 2b, 2c) comprises at least one channel (7) that is fillable with gaseous and/or liquid media.

8. A device according to claim 7, **characterised in that** at least one channel (7) is permeable or semipermeable for releasing the gaseous and/or liquid media.

9. A device according to claim 8, **characterised in that** at least one channel (7, 7a) includes openings (8, 9), for instance openings (8) that are permeable on one side, or openings (9) that are permeable on both sides.

10. A device according to any one of claims 7 to 9, **characterised in that** at least one channel (7, 7a) is impermeable for the gaseous and/or liquid media.

11. A device according to any one of claims 7 to 10, **characterised in that** the channels (7) are disposed at the layer (2a) of the applicator that faces the body part.

12. A device according to any one of claims 7 to 11, **characterised in that** the channels (7) are detachably attached to the layer (2) of the applicator (1).

13. A device according to any one of claims 7 to 12, **characterised in that** the channels (7, 7a) extend inside one another.

14. An arrangement according to any one of claims 1 to 13, **characterised in that** each one of the chamber(s) (3a, 3b, 3c, 3d) is subdivided into additional, mutually communicating sub-chambers.

15. A device according to any one of claims 1 to 14, **characterised in that** the control or regulating means is connected to valves in lines (5) for the gaseous and/or liquid media for regulating the flowthrough volume of the gaseous and/or liquid media.

16. A device according to any one of claims 1 to 15, **characterised in that** the layers (2a, 2b, 2c) of the applicator (1) are produced from flexible material such as stretched polytetrafluoroethylene or polyvinylchloride.

17. A device according to any one of claims 1 to 16, **characterised in that** the applicator (1) and, if desired, also the biosensors of the inventive device are disposed in a dimensionally stable casing that at least partially surrounds the body or parts thereof.

## Revendications

1. Dispositif pour la délivrance de paramètres physico-chimiques à appliquer sur le corps ou des parties de ce dernier, où le dispositif présente un support (1) avec au moins deux couches, et l'espace entre les couches est divisé en au moins deux chambres et/ou canaux, qui peuvent être remplis de milieux gazeux et/ou liquides pour la sortie des paramètres physico-chimiques, et le dispositif présente par ailleurs au moins un dispositif de contrôle ou de réglage pour le contrôle ou le réglage des paramètres, comme par exemple le débit, la température, la pression ou similaire, du milieu se trouvant dans l'espace, qui est relié au support où des biocapteurs pour l'acquisition de paramètres corporels, comme par exemple la température, l'ECG ou similaire, sont reliés au dispositif de contrôle ou de réglage, **caractérisé en ce que** les chambres (3a, 3b, 3c, 3d) et/ou canaux peuvent être remplis indépendamment les uns des autres avec les milieux, et **en ce que** les milieux sont contrôlables ou réglables dans chaque chambre (3a, 3b, 3c, 3d) et/ou canal par le dispositif de contrôle ou de réglage en fonction des paramètres corporels acquis par les biocapteurs.

2. Dispositif selon la revendication 1, **caractérisé en ce que** des ouvertures pouvant être obturées (6) sont prévues entre les chambres (3a, 3b, 3c, 3d).

3. Dispositif selon la revendication 1 ou 2, **caractérisé en ce que** les chambres (3a, 3b, 3c, 3d) ou/et canaux sont disposés les uns à côté des autres et/ou les uns après les autres.

4. Dispositif selon l'une des revendications 1 à 3, **caractérisé en ce qu'**au moins une couche (2a, 2b, 2c) du support (1), de préférence la couche (2a) orientée vers la partie du corps, est perméable ou semi-perméable pour la sortie des milieux gazeux et/ou liquides sur la partie du corps.

5. Dispositif selon la revendication 4, **caractérisé en ce que** la couche (2a) présente des ouvertures (4), des pores, des vannes, du tissu semi-perméable ou similaires.

6. Dispositif selon l'une des revendications 1 à 5, **caractérisé en ce qu'**au moins une couche (2a, 2b, 2c) du support (1), de préférence la couche opposée à la partie du corps (2b, 2c), est imperméable aux milieux se trouvant dans les chambres (3a, 3b, 3c, 3d) ou/et canaux.

7. Dispositif selon l'une des revendications 1 à 6, **caractérisé en ce qu'**au moins une des couches (2a, 2b, 2c) comporte au moins un canal (7) pouvant être rempli de milieu gazeux et/ou liquides.

8. Dispositif selon la revendication 7, **caractérisé en ce qu'**au moins un canal (7) est perméable ou semi-perméable pour la sortie des milieux gazeux et/ou liquides.

9. Dispositif selon la revendication 8, **caractérisé en ce qu'**au moins un canal (7, 7a) présente des ouvertures (8, 9), par exemple des ouvertures perméables sur une face (8) ou des ouvertures perméables sur les deux faces (9).

10. Dispositif selon l'une des revendications 7 à 9, **caractérisé en ce qu'**au moins un canal (7, 7a) est imperméable aux milieux gazeux et/ou liquides.

11. Dispositif selon l'une des revendications 7 à 10, **caractérisé en ce que** les canaux (7) sont disposés sur la couche du support (2a) dirigée vers la partie du corps.

12. Dispositif selon l'une des revendications 7 à 11, **caractérisé en ce que** les canaux (7) sont disposés de manière amovible sur la couche (2) du support (1).

13. Dispositif selon l'une des revendications 7 à 12, **caractérisé en ce que** les canaux (7, 7a) sont disposés l'un dans l'autre.

14. Dispositif selon l'une des revendications 1 à 13, **caractérisé en ce que** la chambre ou les chambres (3a, 3b, 3c, 3d) sont divisées chaque fois en d'autres sous-chambres communiquant les unes avec les autres.

15. Dispositif selon l'une des revendications 1 à 14, **caractérisé en ce que** le dispositif de contrôle ou de réglage est relié à des vannes dans les conduites (5) pour les milieux gazeux et/ou liquides, pour régler le débit des milieux gazeux et/ou liquides.

16. Dispositif selon l'une des revendications 1 à 15, **caractérisé en ce que** les couches (2a, 2b, 2c) du support (1) sont préparées à partir d'un matériau flexible, comme par exemple du polytétrafluoréthylène ou du poly(chlorure de vinyle) étiré.

17. Dispositif selon l'une des revendications 1 à 16, **caractérisé en ce que** le support (1) et, si cela est souhaité, également les biocapteurs du dispositif suivant l'invention sont disposés dans un gainage indéformable, enfermant au moins partiellement le corps ou des parties de celui-ci.
